# EUROPÄISCHE PATENTSCHRIFT

(11) **EP 2 036 934 B1**
(45) Veröffentlichungstag und Bekanntmachung des Hinweises auf die Patenterteilung: **20.04.2011**
(21) Anmeldenummer: 08015439.6
(22) Anmeldetag: 02.09.2008
(51) Int. Cl.: C08G 18/72, C08G 18/78, C08G 18/22, C09D 175/04, C09J 175/04

(54) **Polyisocyanatmischungen**
Polyisocyanate mixtures
Mélanges de polyisocyanate

(30) Priorität: 14.09.2007 DE 102007044034
(43) Veröffentlichungstag der Anmeldung: 18.03.2009
(73) Patentinhaber: Bayer MaterialScience AG, 51368 Leverkusen (DE)
(72) Erfinder: Wamprecht, Christian, Dr., 41472 Neuss (DE)

(56) Entgegenhaltungen:
- EP-A- 1 930 357
- DE-A1- 4 403 233
- FR-A- 2 380 309
- US-A1- 2004 197 570

## Beschreibung

Die Erfindung betrifft flüssige Polyisocyanatmischungen auf Basis 2,4'-Diphenylmethandiisocyanat, ein Verfahren zu ihrer Herstellung und ihre Verwendung in Ein- und Zweikomponenten-Polyurethan Beschichtungsmitteln, Kleb- und Dichtstoffen.

Zweikomponenten-Polyurethanbeschichtungsmittel auf Basis 4,4'-Diphenylmethandiisocyanat sind bekannt. So beschreibt die EP-A 0 666 276 bei Temperaturen oberhalb 5 °C flüssige Polyisocyanatmischungen auf Basis von 4,4'-Diphenylmethan-diisocyanat mit einem Isocyanatgruppengehalt von 14,5 bis 24 Gew.-% und einem Gehalt an Allophanatgruppen (berechnet als C₂HN₂O₃) von 7,7 bis 14,5 Gew.-% sowie ein Verfahren zu deren Herstellung. Diese Polyisocyanatmischungen zeichnen sich durch eine relativ gute Kristallisationsstabilität und eine hohe Reaktivität in Kombination mit Hydroxylgruppen enthaltenden Reaktionspartnern aus. In der einleitenden Beschreibung der EP-A 0 666 276 wird erwähnt, dass Mischungen, bestehend aus 4,4'-Diphenylmethandiisocyanat und größeren Mengen 2,4'-Diphenylmethandiisocyanat zwar flüssig sind, jedoch eine schlechte Kristallisationsstabilität aufweisen und sich die Eigenschaften von daraus hergestellten Beschichtungsmaterialien aufgrund des Anteils an 2,4'-Diphenylmethandiisocyanat deutlich verschlechtern.

Für manche Anwendungen ist jedoch der Einsatz von Polyisocyanaten auf Basis von 4,4'-Diphenylmethandiisocyanat aufgrund der hohen Reaktivität dieses Isocyanats nicht wünschenswert. Vielmehr besteht der Bedarf an Polyisocyanaten, die in entsprechenden Anwendungen ähnliche Materialeigenschaften wie solche auf Basis von 4,4'-Diphenylmethandiisocyanat erhältlichen aufweisen, jedoch eine geringere Reaktivität haben und somit eine längere Verarbeitungszeit ermöglichen.

2,4'-Diphenylmethandiisocyanat hat aufgrund der Isocyanatgruppe in 2-Stellung eine geringere Reaktivität gegenüber isocyanatreaktiven Reaktionspartnern als 4,4'-Diphenylmethandiisocyanat, allerdings ist 2,4'-Diphenylmethandiisocyanat bei Raumtemperatur fest und auch Polyisocyanate auf Basis von 2,4'-Diphenylmethandiisocyanat weisen eine schlechte Kristallisationsstabilität und zumeist auch hohe Viskositäten auf.

Aufgabe der Erfindung war daher die Bereitstellung von neuen Polyisocyanatgemischen, die im Vergleich zu solchen auf Basis von 4,4'-Diphenylmethandiisocyanat eine geringere Reaktivität und zu solchen auf Basis von 2,4'-Diphenylmethandiisocyanat eine deutlich bessere Kristallisationsstabilität aufweisen, selbst bei tiefen Temperaturen von unterhalb 5 °C. Diese Aufgabe konnte durch Bereitstellung der nachstehend näher charakterisierten Allophanatgruppen aufweisenden Polyisocyanatgemische gelöst werden.

Hier und im Folgenden steht 2,4'-Diphenylmethandiisocyanat sowohl für das reine 2,4'-Isomer als auch für dessen Gemische mit bis zu 2,0% an 4,4'- und/oder bis zu 1,0% an 2,2'-Diphenylmethandiisocyanat, wobei sich die Prozentangaben jeweils auf das Gesamtgemisch beziehen. Vorzugsweise besteht das als Ausgangsmaterial eingesetzte 2,4'-Diphenylmethandiisocyanat zumindest zu 98,5% aus dem 2,4'-Isomeren.

Gegenstand der Erfindung sind bei Temperaturen von oberhalb -5 °C flüssige Polyisocyanatmischungen auf Basis von 2,4'-Diphenylmethandiisocyanat, gekennzeichnet durch
A) einen NCO-Gehalt von 14 bis 24 Gew.-% und
B) einen Gehalt an Allophanatgruppen (berechnet als C₂HN₂O₃, Molekulargewicht = 101) von 7,7 bis 17,6 Gew.-%.

Gegenstand der Erfindung ist auch ein Verfahren zur Herstellung dieser Polyisocyanatmischungen, welches dadurch gekennzeichnet ist, dass man 2,4'-Diphenylmethandiisocyanat unter intermediärer Urethanbildung mit einwertigen Alkoholen mit 3 bis 18 Kohlenstoffatomen pro Molekül unter Einhaltung eines NCO/OH-Verhältnisses von 4:1 bis 8,5:1 bei bis zu 160 °C liegenden Temperaturen zur Reaktion bringt, wobei man die Umsetzung spätestens nach erfolgter Urethanbildung in Gegenwart eines die Allophanatbildung fördernden Katalysators durchführt.

Gegenstand der Erfindung ist schließlich auch die Verwendung der erfindungsgemäßen Polyisocyanatgemische als Polyisocyanatkomponente in Ein- und Zweikomponenten-Polyurethan Beschichtungsmitteln, Klebstoffen und Dichtstoffen, sowie als Rohstoffe bei der Herstellung von isocyanatgruppenhaltigen Polyisocyanaten, insbesondere von Prepolymeren, sowie zur Synthese von Polyurethandispersionen und Polyurethanlösungen.

Allophanatgruppen aufweisende Polyisocyanatgemische sind bereits bekannt und beispielsweise in US-A 3 769 318, GB-A 994 890 oder EP-A 0 666 276 beschrieben. Obwohl 4,4'-Diphenylmethandiisocyanat als geeignetes Ausgangsmaterial genannt wird, kann gesagt werden, dass diesen Veröffentlichungen kein Hinweis darauf zu entnehmen ist, ob und unter welchen Bedingungen flüssige, kristallisationsstabile Polyisocyanatmischungen durch Modifizierung von 2,4'-Diphenylmethandiisocyanat hergestellt werden können.

Ausgangmaterial für das erfindungsgemäße Verfahren sind neben 2,4'-Diphenylmethandiisocyanat einwertige Alkohole mit 3 bis 18 Kohlenstoffatomen pro Molekül. Bevorzugt werden lineare, d. h. unverzweigte, bei Raumtemperatur flüssige, einwertige aliphatische Alkohole mit 3 bis 10 Kohlenstoffatomen eingesetzt. Mischungen verschiedener derartiger Alkohole können selbstverständlich ebenfalls verwendet werden.

Geeignete Alkohole sind beispielsweise n-Propanol, i-Propanol, n-Butanol, i-Butanol, n-Pentanol, n-Hexanol, n-Octanol, 2-Ethylhexanol, n-Decanol, n-Dodecanol, n-Hexadecanol, aber auch ethergruppenhaltige Alkohole, einwertige Alkohole, wie sie durch Ethoxylierung oder Propoxylierung der beispielhaft genannten einfachen Alkanole erhalten werden, wie z. B. Ethylenglykolmonomethylether, Ethylenglykolmonoethylether, Ethylenglykolmonobutylether, Ethylenglykolmonoisopropylether, Diethylenglykolmonobutylether. Bevorzugt sind ethergruppenfreie Monoalkohole.

Die Allophanatisierung erfolgt zumindest im Anschluss an die intermediäre Urethanbildung in Gegenwart von die Allophanatisierung fördernden Katalysatoren. Geeignete Katalysatoren sind beispielsweise in dem Reaktionsgemisch lösliche Metallverbindungen der 3. und 4. Hauptgruppe, sowie 1., 2., 6., 7. und 8. Nebengruppe der in US-A 3 769 318 genannten Art. Besonders bevorzugt werden Zinn(II)octoat oder Zinkacetylacetonat in Mengen von 20 bis 2000 ppm (Gewicht), vorzugsweise 20 bis 200 ppm (Gewicht), bezogen auf das Reaktionsgemisch eingesetzt. Die Anwesenheit von alkylierend wirkenden Verbindungen während der Reaktion ist im Unterschied zur Empfehlung der UP-A 3 769 318 nicht erforderlich. Die erfindungsgemäßen Polyisocyanatmischungen enthalten ausschließlich Allophanatstrukturen und keine Urethan-, Isocyanurat und/oder Carbodiimidstrukturen. Dies wird durch 13C-NMR-spektroskopische Untersuchungen zweifelsfrei belegt.

Das erfindungsgemäße Verfahren wird bei Temperaturen von bis zu 160 °C durchgeführt. Die bevorzugte Temperatur bezüglich der Allophanatisierung liegt bei 80 bis 120 °C. Gemäß einer bevorzugten Arbeitsweise werden die erfindungsgemäßen Polyisocyanatgemische in einer 2-stufigen Verfahrensweise hergestellt. In einem ersten Verfahrensschritt wird geschmolzenes 2,4'-Diphenylmethandiisocyanat vorgelegt und bei einer Temperatur zwischen 40 und 80 °C, vorzugsweise zwischen 50 und 70 °C der Monoalkohol zugetropft. Nach Erreichen des für die Urethanbildung berechneten NCO-Gehaltes wird im zweiten Verfahrensschritt der Katalysator zugesetzt und anschließend die Temperatur auf 80 bis 160 °C, vorzugsweise auf 80 bis 120 °C erhöht. Anschließend wird die Reaktion bei Erreichen des für die Allophanatisierung berechneten NCO-Gehaltes, vorzugsweise durch Zugabe eines Katalysatorgiftes, abgebrochen. Geeignete Katalysatorgifte sind beispielsweise alkylierend oder acylierend wirkende Verbindungen, wie z. B. p-Toluolsulfonsäuremethylester, Dimethylsulfat, Benzoylchlorid oder Isophthaloylchlorid, die vorzugsweise in mindestens äquimolaren Mengen, bezogen auf die Menge an verwendetem Katalysator eingesetzt werden.

Die erfindungsgemäßen Polyisocyanatgemische sind fast farblose bis dunkel gelb gefärbte flüssige Gemische mit niedrigen Viskositäten zwischen 200 und 80.000 mPa·s (23 °C), wobei die Viskosität mit steigender Menge an Allophanatstrukturen zunimmt. Dabei zeigt es sich, dass die Viskositäten überraschenderweise niedriger sind, als die der entsprechenden 4,4'-MDI-Allophanate. Das ist sehr überraschend, weil die Viskosität von reinem 2,4'-MDI bei 40 °C mit 11,0 mPa·s höher liegt als die von reinem 4,4'-MDI (4,1 mPa·s). Der NCO-Gehalt der erfindungsgemäßen Polyisocyanatgemische liegt bei 14 bis 24 Gew.-%.

Die erfindungsgemäßen Polyisocyanatgemische zeigen im Vergleich zu entsprechenden Gemischen auf Basis von 4,4'-Diphenylmethandiisocyanat eine verbesserte Kristallisationsbeständigkeit bei tiefen Temperaturen, z. B. bei unter 5 °C. Produktgemische mit einem hohen Allophanatisierungsgrad zeigen sogar bei Temperaturen unter -5 °C eine gute Kristallisationsbeständigkeit.

Die erfindungsgemäßen Polyisocyanatmischungen sind geeignete Ausgangsverbindungen zur Herstellung von Polyurethankunststoffen im allgemeinen. Bevorzugt werden die Gemische jedoch zur Herstellung lösemittelfreier Polyurethanbeschichtungen, Polyurethandichtstoffe und Polyurethanklebstoffe, für Polyharnstoffbeschichtungen sowie zur Synthese von Polyurethandispersionen und Polyurethanlösungen eingesetzt. Zur Herstellung dieser Produkte werden die erfindungsgemäßen Polyisocyanatgemische mit aus der Polyurethanchemie an sich bekannten, lösemittelfreien Polyhydroxylverbindungen bzw. Gemische derselben kombiniert und zur Reaktion gebracht, wobei vorzugsweise die Mengenverhältnisse der Einzelkomponenten einem NCO/OH-Äquivalentverhältnis von 0,8 : 1 bis 5 : 1, vorzugsweise 0,8 : 1 bis 3 : 1 und besonders bevorzugt 0,8 : 1 bis 1,5 : 1 entsprechen. Geeignete Polyhydroxylverbindungen sind beispielsweise Polyether-, Polyester-, Polyacrylat- und Polycarbonatpolyole. Um eine blasenfreie Aushärtung bei Beschichtungsanwendungen mit hohen Schichtstärken zu gewährleisten werden die erfindungsgemäßen Polyisocyanatmischungen bevorzugt mit Rizinusöl bzw. Abmischungen von Rizinusöl mit Keton-Formaldehydharzen und/oder Polyesterpolyolen und/oder Polyetherpolyolen und/oder Polycarbonatpolyolen kombiniert. Solche Prepolymeren auf Basis von 2,4'-MDI-Allophanaten zeichnen sich durch eine bessere Lagerbeständigkeit und eine längere Verarbeitungszeit im Vergleich zu Systemen auf Basis 4,4'-MDI aus.

Ein weiterer Anwendungsbereich sind Polyurethandispersionen, bei denen die 2,4'-MDI-Allophanate aufgrund ihrer geringeren Reaktivität im Vergleich zu 4,4'-Allophanaten eine geringere Tendenz zu unerwünschten Nebenreaktionen, z. B. mit Carbonsäuregruppen und Wasser, zeigen.

Für Klebstoff- und Dichtstoffanwendungen werden die erfindungsgemäßen Polyisocyanatmischungen bevorzugt mit Polyesterpolyolen und/oder Polyetherpolyolen kombiniert. Im Falle der Herstellung von wässrigen Polyurethandispersionen auf Basis der erfindungsgemäßen Polyisocyanatmischungen kommen vorzugsweise Polyesterpolyole und/oder Polycarbonatpolyole als Reaktionspartner zum Einsatz. Polyacrylatpolyole werden als Reaktionspartner für die erfindungsgemäßen Polyisocyanatmischungen dann bevorzugt, wenn Beschichtungsanwendungen mit geringen Schichtstärken und schneller physikalischer Trocknung erforderlich sind.

Der für die meisten Anwendungen erforderliche Zusatz von Pigmenten und anderen Hilfs- und Zusatzmitteln, wie Füllstoffen, Verlaufshilfsmitteln wird vorzugsweise so vorgenommen, dass diese Zusatzmittel in eine der Ausgangkomponenten eingemischt werden. Die die erfindungsgemäßen Polyisocyanatmischungen enthaltenden Produkte können nach den üblichen bekannten Methoden, wie beispielsweise durch Spritzen, Streichen, Tauchen, Fluten, oder mit Hilfe von Walzen oder Rakeln auf beliebige Substrate ein- oder mehrschichtig aufgetragen werden.

Als Substrate eignen sich beispielsweise Metall, Holz, Glas, Stein, keramische Materialien, Beton, harte und flexible Kunststoffe, Textilien, Leder oder Papier. Die Substrate können vor der Applikation mit den üblichen Grundierungen versehen werden.

Die nachfolgenden Beispiele dienen zur weiteren Erläuterung der Erfindung. Alle Prozentangaben beziehen sich auf das Gewicht. Das in den nachfolgenden Beispielen eingesetzte 2,4'-Diphenylmethandiisocyanat stellt ein technisches Produkt mit einem Gehalt von 99,25 Gew.-% des 2,4'-Isomeren dar.

### Beispiele:

### Beispiel 1

### Herstellung einer erfindungsgemäßen Polyisocyanatmischung

931 g geschmolzenes 2,4'-Diphenylmethandiisocyanat werden in einem 2 Liter Dreihalskolben mit Rührer, Rückflusskühler und Tropftrichter unter Stickstoffatmosphäre vorgelegt und auf 60 °C erwärmt. Bei dieser Temperatur werden 69 g n-Butanol (NCO/OH-Verhältnis 8:1) innerhalb von 20 Minuten zugetropft. Danach wird so lange bei 60 °C gerührt, bis der für die Urethanbildung berechnete Isocyanatgehalt von 27,4 % erreicht ist. Danach werden 0,1 g Zinkacetylacetonat zugegeben und die Temperatur auf 100 °C erhöht. Bei dieser Temperatur wird so lange gerührt, bis der für die Allophanatbildung berechnete NCO-Gehalt von 23,5 % erreicht ist. Danach wird die Reaktion durch Zugabe von 0,1 g Benzoylchlorid beendet. Anschließend wird auf 30 °C abgekühlt. Man erhält ein klares, gelblich gefärbtes Polyisocyanatgemisch mit einer Viskosität von 250 mPa·s (23 °C). Das Produkt ist bei einer Temperatur von 0 °C 2 Wochen kristallisationsstabil, bei einer Temperatur von - 10 °C trübt das Produkt nach ca. 6 Tagen ein. Das Produkt enthält einen berechneten Anteil an Allophanatgruppen von 9,4 %.

### Beispiel 2

### Herstellung einer erfindungsgemäßen Polyisocyanatmischung

9218 g geschmolzenes 2,4'-Diphenylmethandiisocyanat werden in einem 15 Liter Metallreaktor mit Rührer, Rückflusskühler und Tropftrichter unter Stickstoffatmosphäre vorgelegt und auf 60 °C erwärmt. Bei dieser Temperatur werden 780 g n-Butanol (NCO/OH-Verhältnis 7:1) innerhalb von 20 Minuten zugetropft. Danach wird so lange bei 60 °C gerührt, bis der für die Urethanbildung berechnete Isocyanatgehalt von 26,5 % erreicht ist. Danach werden 1,0 g Zinkacetylacetonat zugegeben und die Temperatur auf 100 °C erhöht. Bei dieser Temperatur wird so lange gerührt, bis der für die Allophanatbildung berechnete NCO-Gehalt von 22,1 % erreicht ist. Danach wird die Reaktion durch Zugabe von 1,0 g Benzoylchlorid beendet. Anschließend wird auf 30 °C abgekühlt. Man erhält ein klares, gelblich gefärbtes Polyisocyanatgemisch mit einer Viskosität von 795 mPa·s (23 °C). Das Produkt ist bei einer Temperatur von 0 °C 3 Wochen kristallisationsstabil, bei einer Temperatur von - 10 °C trübt das Produkt nach ca. 10 Tagen ein. Das Produkt enthält einen berechneten Anteil an Allophanatgruppen von 10,6 %.

### Beispiel 3

### Herstellung einer erfindungsgemäßen Polyisocyanatmischung

9100 g geschmolzenes 2,4'-Diphenylmethandiisocyanat werden in einem 15 Liter Metallreaktor mit Rührer, Rückflusskühler und Tropftrichter unter Stickstoffatmosphäre vorgelegt und auf 60 °C erwärmt. Bei dieser Temperatur werden 898 g n-Butanol (NCO/OH-Verhältnis 6:1) innerhalb von 20 Minuten zugetropft. Danach wird so lange bei 60 °C gerührt, bis der für die Urethanbildung berechnete Isocyanatgehalt von 25,5 % erreicht ist. Danach werden 1,0 g Zinkacetylacetonat zugegeben und die Temperatur auf 100 °C erhöht. Bei dieser Temperatur wird so lange gerührt, bis der für die Allophanatbildung berechnete NCO-Gehalt von 20,4 % erreicht ist. Danach wird die Reaktion durch Zugabe von 1,0 g Benzoylchlorid beendet. Anschließend wird auf 30 °C abgekühlt. Man erhält ein klares, gelblich gefärbtes Polyisocyanatgemisch mit einer Viskosität von 2930 mPa·s (23 °C). Das Produkt ist bei einer Temperatur von 0 °C 4 Wochen kristallisationsstabil, bei einer Temperatur von - 10 °C trübt das Produkt nach ca. 15 Tagen ein. Das Produkt enthält einen berechneten Anteil an Allophanatgruppen von 12,2 %.

### Beispiel 4

### Herstellung einer erfindungsgemäßen Polyisocyanatmischung

871 g geschmolzenes 2,4'-Diphenylmethandiisocyanat werden in einem 2 Liter Dreihalskolben mit Rührer, Rückflusskühler und Tropftrichter unter Stickstoffatmosphäre vorgelegt und auf 60 °C erwärmt. Bei dieser Temperatur werden 129 g n-Butanol (NCO/OH-Verhältnis 4:1) innerhalb von 20 Minuten zugetropft. Danach wird so lange bei 60 °C gerührt, bis der für die Urethanbildung berechnete Isocyanatgehalt von 21,9 % erreicht ist. Danach werden 0,2 g Zinkacetylacetonat zugegeben und die Temperatur auf 100 °C erhöht. Bei dieser Temperatur wird so lange gerührt, bis der für die Allophanatbildung berechnete NCO-Gehalt von 14,6 % erreicht ist. Danach wird die Reaktion durch Zugabe von 0,2 g Benzoylchlorid beendet. Anschließend wird auf 30 °C abgekühlt. Man erhält ein klares, gelblich gefärbtes Polyisocyanatgemisch mit einer Viskosität von 71550 mPa·s (23 °C). Das Produkt ist bei einer Temperatur von 0 °C 2 Monate kristallisationsstabil, bei einer Temperatur von - 10 °C trübt das Produkt nach ca. 3 Wochen ein. Das Produkt enthält einen berechneten Anteil an Allophanatgruppen von 17,6 %.

### Beispiel 5

### Herstellung einer erfindungsgemäßen Polyisocyanatmischung

943 g geschmolzenes 2,4'-Diphenylmethandiisocyanat werden in einem 2 Liter Dreihalskolben mit Rührer, Rückflusskühler und Tropftrichter unter Stickstoffatmosphäre vorgelegt und auf 60 °C erwärmt. Bei dieser Temperatur werden 57 g 2-Propanol (NCO/OH-Verhältnis 8:1) innerhalb von 20 Minuten zugetropft. Danach wird so lange bei 60 °C gerührt, bis der für die Urethanbildung berechnete Isocyanatgehalt von 27,7 % erreicht ist. Danach werden 0,1 g Zinkacetylacetonat zugegeben und die Temperatur auf 100 °C erhöht. Bei dieser Temperatur wird so lange gerührt, bis der für die Allophanatbildung berechnete NCO-Gehalt von 23,7 % erreicht ist. Danach wird die Reaktion durch Zugabe von 0,1 g Benzoylchlorid beendet. Anschließend wird auf 30 °C abgekühlt. Man erhält ein klares, gelblich gefärbtes Polyisocyanatgemisch mit einer Viskosität von 718 mPa·s (23 °C). Das Produkt ist bei einer Temperatur von 0 °C 2 Wochen kristallisationsstabil, bei einer Temperatur von - 10 °C trübt das Produkt nach ca. 6 Tagen ein. Das Produkt enthält einen berechneten Anteil an Allophanatgruppen von 9,5 %.

### Beispiel 6

### Herstellung einer erfindungsgemäßen Polyisocyanatmischung

894 g geschmolzenes 2,4'-Diphenylmethandiisocyanat werden in einem 2 Liter Dreihalskolben mit Rührer, Rückflusskühler und Tropftrichter unter Stickstoffatmosphäre vorgelegt und auf 60 °C erwärmt. Bei dieser Temperatur werden 106 g n-Butanol (NCO/OH-Verhältnis 5:1) innerhalb von 20 Minuten zugetropft. Danach wird so lange bei 60 °C gerührt, bis der für die Urethanbildung berechnete Isocyanatgehalt von 24,0 % erreicht ist. Danach werden 0,2 g Zinkacetylacetonat zugegeben und die Temperatur auf 100 °C erhöht. Bei dieser Temperatur wird so lange gerührt, bis der für die Allophanatbildung berechnete NCO-Gehalt von 18,0 % erreicht ist. Danach wird die Reaktion durch Zugabe von 0,2 g Benzoylchlorid beendet. Anschließend wird auf 30 °C abgekühlt. Man erhält ein klares, gelblich gefärbtes Polyisocyanatgemisch mit einer Viskosität von 6580 mPa·s (23 °C). Das Produkt ist bei einer Temperatur von 0 °C 2 Monate kristallisationsstabil, bei einer Temperatur von - 10 °C trübt das Produkt nach ca. 3 Wochen ein. Das Produkt enthält einen berechneten Anteil an Allophanatgruppen von 14,4 %.

## Patentansprüche

1. Bei Temperaturen von oberhalb -5 °C flüssige Polyisocyanatmischungen auf Basis von 2,4'-Diphenylmethandiisocyanat, **gekennzeichnet durch**
A) einen NCO-Gehalt von 14,5 bis 24 Gew.-%,
B) einen Gehalt von in 2-Stellung gebundenen Isocyanatgruppen von > 50 % und
C) einen Gehalt an Allophanatgruppen (berechnet als C₂HN₂O₃, Molekulargewicht = 101) von 7,7 bis 17,6 Gew.-%,
wobei 2,4'-diphenylmethandiisocyanat sowohl für das eine 2,4-isomer als auch für dessen gemische mit bis zu 2,0% an 4,4'- und/oder bis zu 1,0% an 2,2-diphenylemethandiisocyanat stallt.

2. Polyisocyanatmischungen gemäß Anspruch 1, **dadurch gekennzeichnet, dass** sie unter intermediärer Urethanbildung hergestellte Umsetzungsprodukte von
A) 2,4'-Diphenylmethandiisocyanat mit
B) einwertigen Alkoholen mit 3 bis 18 Kohlenstoffatomen pro Molekül unter Einhaltung eines NCO/OH-Verhältnisses von 4:1 bis 8,5:1 umfassen,
wobei 2,4'-diphenylmethandiisocyanat sowohl für das eine 2,4-isomer als auch für dessen gemische mit bis zu 2,0% an 4,4'- und/oder bis zu 1,0% an 2,2-diphenylemethandiisocyanat stallt.

3. Polyisocyanatmischungen gemäß Anspruch 1, **dadurch gekennzeichnet, dass** sie unter intermediärer Urethanbildung hergestellte Umsetzungsprodukte von
A) 2,4'-Diphenylmethandiisocyanat mit
B) linearen, einwertigen Alkoholen mit 4 bis 10 Kohlenstoffatomen
umfassen,
wobei 2,4'-diphenylmethandiisocyanat sowohl für das eine 2,4-isomer als auch für dessen gemische mit bis zu 2,0% an 4,4'- und/oder bis zu 1,0% an 2,2-diphenylemethandiisocyanat stallt.

4. Verfahren zur Herstellung von bei tiefen Temperaturen zwischen -5 und +5 °C flüssigen Polyisocyanatmischungen auf Basis von 2,4'-Diphenylmethandiisocyanat, **dadurch gekennzeichnet, dass** man 2,4'-Diphenylmethandiisocyanat unter intermediärer Urethanbildung mit einwertigen Alkoholen mit 3 bis 18 Kohlenstoffatomen pro Molekül unter Einhaltung eines NCO/OH-Verhältnisses von 4:1 bis 8,5:1 bei bis zu 160 °C liegenden Temperaturen zur Reaktion bringt, wobei man die Umsetzung spätestens nach erfolgter Urethanbildung in Gegenwart eines die Allophanatbildung fördernden Katalysators durchführt,
wobei 2,4'-diphenylmethandiisocyanat sowohl für das eine 2,4-isomer als auch für dessen gemische mit bis zu 2,0% an 4,4'- und/oder bis zu 1,0% an 2,2-diphenylemethandiisocyanat stallt.

5. Verfahren gemäß Anspruch 4, **dadurch gekennzeichnet, dass** man als die Allophanatisierung fördernde Katalysatoren in dem Reaktionsgemisch lösliche Verbindungen der dritten oder vierten Hauptgruppe oder der 1., 2., 6., 7. oder 8. Nebengruppe des Periodensystems der Elemente verwendet.

6. Verfahren gemäß Ansprüchen 4 und 5, **dadurch gekennzeichnet, dass** man als Katalysator Zinn(II)octoat oder Zinkacetylaeetonat verwendet.

7. Verwendung der Polyisocymatrnischungen gemäß Ansprüchen 1 bis 3 als Polyisocyanatkomponente zur Herstellung von isocyanatfunktionellen Prepolymeren, von Polyurethandispersionen, Polyurcthanlösungen und zur Herstellung von silanterminierten Prepolymeren.

8. Verwendung der Polyisocyanatmischungen gemäß Ansprüchen 1 bis 3 als Polyisocyanatkomponente in Ein- und Zweikomponenten-Polyurethan-Beschiehtungsmitteln.

9. Klebstoffe enthaltend unter Verwendung von Polyisocyanatmischungen gemäß Ansprüchen 1 bis 3 erhältliche Verbindungen.

10. Dichtstoffe enthaltend unter Verwendung von Polyisocyanatmischungen gemäß Ansprüchen 1 bis 3 erhältliche Verbindungen.

## Claims

1. Polyisocyanate mixtures which are liquid at temperatures above -5°C and are based on diphenylmethane 2,4'-diisocyanate, **characterized by**
A) an NCO content of 14.5 to 24% by weight,
B) a content of isocyanate groups bonded in the 2 position of > 50% and
C) a content of allophanate groups(calculated as C₂HN₂O₃, molecular weight = 101) of 7.7 to 17.6% by weight,
where 2,4'-diphenylmethane diisocyanate represents either the pure 2,4' isomer or mixtures thereof with up to 2.0% of 4,4'- and/or up to 1.0% of 2,2'-diphenylmethane diisocyanate.

2. Polyisocyanate mixtures according to Claim 1, **characterized in that** they comprise reaction products prepared with intermediate urethane formation from
A) diphenylmethane 2,4'-diisocyanate with
B) monohydric alcohols having 3 to 18 carbon atoms per molecule while maintaining an NCO/OH ratio of 4:1 to 8.5:1,
where 2,4'-diphenylmethane diisocyanate represents either the pure 2,4' isomer or mixtures thereof with up to 2.0% of 4,4'- and/or up to 1.0% of 2,2'-diphenylmethane diisocyanate.

3. Polyisocyanate mixtures according to Claim 1, **characterized in that** they comprise reaction products prepared with intermediate urethane formation from
A) diphenylmethane 2,4'-diisocyanate with
B) linear, monohydric alcohols having 4 to 10 carbon atoms,
where 2,4'-diphenylmethane diisocyanate represents either the pure 2,4' isomer or mixtures thereof with up to 2.0% of 4,4'- and/or up to 1.0% of 2,2'-diphenylmethane diisocyanate.

4. Process for preparing polyisocyanate mixtures which are liquid at low temperatures between -5 and +5°C and are based on diphenylmethane 2,4'-diisocyanate, **characterized in that** diphenylmethane 2,4'-diisocyanate is reacted with monohydric alcohols having 3 to 18 carbon atoms per molecule while maintaining an NCO/OH ratio of 4:1 to 8.5:1 at temperatures up to 160°C with intermediate urethane formation, the reaction being carried out no later than after completion of urethane formation in the presence of a catalyst which promotes allophanate formation,
where 2,4'-diphenylmethane diisocyanate represents either the pure 2,4' isomer or mixtures thereof with up to 2.0% of 4,4'- and/or up to 1.0% of 2,2'-diphenylmethane diisocyanate.

5. Process according to Claim 4, **characterized in that** the allophanatization-promoting catalysts used comprise compounds of the third or fourth main group or of transition group 1, 2, 6, 7 or 8 of the Periodic Table of the Elements, which are soluble in the reaction mixture.

6. Process according to Claims 4 and 5, **characterized in that** the catalyst used comprises tin(II) octoate or zinc acetylacetonate.

7. Use of the polyisocyanate mixtures according to Claims 1 to 3 as polyisocyanate component for preparing isocyanate-functional prepolymers, polyurethane dispersions or polyurethane solutions, and for preparing silane-terminated prepolymers.

8. Use of the polyisocyanate mixtures according to Claims 1 to 3 as polyisocyanate component in single- and two-component polyurethane coating compositions.

9. Adhesive comprising compounds obtainable using polyisocyanate mixtures according to Claims 1 to 3.

10. Sealants comprising compounds obtainable using polyisocyanate mixtures according to Claims 1 to 3.

## Revendications

1. Mélanges de polyisocyanates liquides à des températures supérieures à -5°C à base de 2,4'-diphénylméthanediisocyanate, **caractérisés par**
A) une teneur en NCO de 14,5 à 24% en poids,
B) une teneur en groupes isocyanate liés en 2ème position > 50% et
C) une teneur en groupes allophanate (calculés comme C₂HN₂O₃, poids moléculaire = 101) de 7,7 à 17,6% en poids,
où 2,4'-diphénylméthanediisocyanate représente tant l'isomère 2,4' pur que ses mélanges avec jusqu'à 2,0% de 4,4'-diphénylméthanediisocyanate et/ou jusqu'à 1,0% de 2,2'-diphénylméthanediisocyanate.

2. Mélanges de polyisocyanates selon la revendication 1, **caractérisés en ce qu'**ils contiennent des produits de transformation, préparés avec une formation intermédiaire d'uréthane, de
A) 2,4'-diphénylméthanediisocyanate avec
B) des alcools monovalents comprenant 3 à 18 atomes de carbone par molécule
en maintenant un rapport NCO/OH de 4:1 à 8,5:1, où 2,4'-diphénylméthanediisocyanate représente tant l'isomère 2,4' pur que ses mélanges avec jusqu'à 2,0% de 4,4'-diphénylméthanediisocyanate et/ou jusqu'à 1,0% de 2,2'-diphénylméthanediisocyanate.

3. Mélanges de polyisocyanates selon la revendication 1, **caractérisés en ce qu'**ils contiennent des produits de transformation, préparés avec une formation intermédiaire d'uréthane, de
A) 2,4'-diphénylméthanediisocyanate avec
B) des alcools linéaires, monovalents comprenant 4 à 10 atomes de carbone,
où 2,4'-diphénylméthanediisocyanate représente tant l'isomère 2,4' pur que ses mélanges avec jusqu'à 2,0% de 4,4'-diphénylméthanediisocyanate et/ou jusqu'à 1,0% de 2,2'-diphénylméthanediisocyanate.

4. Procédé pour la préparation de mélanges de polyisocyanates liquides à des températures basses entre -5°C et +5°C à base de 2,4'-diphénylméthanediisocyanate, **caractérisé en ce qu'**on fait réagir du 2,4'-diphénylméthanediisocyanate, avec formation intermédiaire d'uréthane, avec des alcools monovalents comprenant 3 à 18 atomes de carbone par molécule, en maintenant un rapport NCO/OH de 4:1 à 8,5:1, à des températures allant jusqu'à 160°C, en réalisant la transformation au plus tard après la fin de la formation de l'uréthane, en présence d'un catalyseur favorisant la formation d'allophanate, où 2,4'-diphénylméthanediisocyanate représente tant l'isomère 2,4' pur que ses mélanges avec jusqu'à 2,0% de 4,4'-diphénylméthanediisocyanate et/ou jusqu'à 1,0% de 2,2'-diphénylméthanediisocyanate.

5. Procédé selon la revendication 4, **caractérisé en ce qu'**on utilise, comme catalyseurs favorisant l'allophanatisation, des composés solubles dans le mélange réactionnel du troisième ou quatrième groupe principal ou du 1er, 2ème, 6ème, 7ème ou 8ème groupe secondaire du système périodique des éléments.

6. Procédé selon les revendications 4 et 5, **caractérisé en ce qu'**on utilise comme catalyseur l'octoate d'étain (II) ou l'acétylacétonate de zinc.

7. Utilisation des mélanges de polyisocyanates selon les revendications 1 à 3 comme composant polyisocyanate destiné à la préparation de prépolymères à fonctionnalité isocyanate, de dispersions de polyuréthane, de solutions de polyuréthane et à la préparation de prépolymères terminés par silane.

8. Utilisation des mélanges de polyisocyanates selon les revendications 1 à 3 comme composant polyisocyanate dans les agents de revêtement à base de polyuréthane à un et deux composants.

9. Adhésifs contenant des composés pouvant être obtenus en utilisant des mélanges de polyisocyanates selon les revendications 1 à 3.

10. Matériaux d'étanchéité contenant des composés pouvant être obtenus en utilisant des mélanges de polyisocyanates selon les revendications 1 à 3.
